# EUROPEAN PATENT APPLICATION

(11) **EP 4 806 477 A1**
(43) Date of publication of application: **16.09.2026**
(21) Application number: 24896974.3
(22) Date of filing: 23.07.2024
(51) Int. Cl.: A61M 5/142

(54) **LIQUID MEDICINE ADMINISTRATION DEVICE AND CONTROL METHOD THEREFOR, LIQUID MEDICINE ADMINISTRATION SYSTEM, AND PROGRAM**

(30) Priority: 01.12.2023 JP 2023204208
(71) Applicant: Terumo Kabushiki Kaisha, Tokyo 151-0072 (JP)
(72) Inventor: YAKUSHIJI Yusuke, Ashigarakami-gun, Kanagawa 259-0151 (JP)
(74) Representative: TBK
(86) International application number: PCT/JP2024/026337
(87) International publication number: WO 2025/115282

(57) **Abstract**

A liquid medicine administration device includes a pump body that administers a liquid medicine filled in a reservoir into a living body of a patient, in which the pump body includes a pump that transfers the liquid medicine filled in the reservoir into the living body of the patient, a rechargeable battery that supplies power for driving the pump, a measurement unit that measures a remaining amount of the rechargeable battery, a detection unit that detects whether or not the pump body is attached to a cradle device to be stuck to a skin of the patient, a notification unit that notifies a user of information, and a control unit, and the control unit causes the notification unit to provide a notification of the remaining amount of the rechargeable battery measured by the measurement unit in a case where the detection unit has detected insertion of the pump body into the cradle device or removal of the pump body from the cradle device.

## Description

### Technical Field

The present disclosure relates to a liquid medicine administration device, a control method therefor, a liquid medicine administration system, and a program.

### Background Art

There is a liquid medicine administration device that administers a liquid medicine such as insulin into a body of a patient. Patent Literature 1 discloses that a battery is provided in a disposable portion and/or a reusable portion and used as a power source.

### Citation List

### Patent Literature

Patent Literature 1: JP 2014-039846 A

### Summary of Invention

### Technical Problem

However, the conventional configuration has room for improvement in effectively notifying a user of a remaining amount of the rechargeable battery of the liquid medicine administration device.

An object of the present disclosure is to make it possible to more effectively notify a user of a remaining amount of a rechargeable battery of a liquid medicine administration device.

### Solution to Problem

According to the present disclosure, there is provided
(1) a liquid medicine administration device including a pump body that administers a liquid medicine filled in a reservoir into a living body of a patient, in which
   the pump body includes
   a pump that transfers the liquid medicine filled in the reservoir into the living body of the patient,
   a rechargeable battery that supplies power for driving the pump,
   a measurement unit that measures a remaining amount of the rechargeable battery,
   a detection unit that detects whether or not the pump body is attached to a cradle device to be stuck to a skin of the patient,
   a notification unit that notifies a user of information, and
   a control unit, and
   the control unit causes the notification unit to provide a notification of the remaining amount of the rechargeable battery measured by the measurement unit in a case where the detection unit has detected insertion of the pump body into the cradle device or removal of the pump body from the cradle device.
(2) In the liquid medicine administration device of (1),
   the control unit may
   determine whether or not a predetermined time has elapsed from previous charging of the rechargeable battery in response to detection of the insertion of the pump body into the cradle device, and
   cause the notification unit to provide the notification of the remaining amount of the rechargeable battery measured by the measurement unit in a case where it is determined that the predetermined time has elapsed from the previous charging of the rechargeable battery.
(3) In the liquid medicine administration device of (1) or (2),
   the control unit may
   determine whether or not the remaining amount of the rechargeable battery measured by the measurement unit is a predetermined threshold or more in response to detection of the removal of the pump body from the cradle device, and
   cause the notification unit to provide the notification of the remaining amount of the rechargeable battery measured by the measurement unit in a case where it is determined that the remaining amount of the rechargeable battery is not the predetermined threshold or more.
(4) In the liquid medicine administration device according to any one of (1) to (3),
   the control unit may
   cause the measurement unit to measure the remaining amount of the rechargeable battery at a predetermined sampling timing, and
   cause the notification unit to provide the notification of the remaining amount of the rechargeable battery measured by the measurement unit in a case where the remaining amount of the rechargeable battery measured at the sampling timing is less than a predetermined threshold.
(5) In the liquid medicine administration device according to any one of (1) to (4),
   the control unit may
   determine whether or not the rechargeable battery is being charged, and
   cause the notification unit to provide the notification of the remaining amount of the rechargeable battery measured by the measurement unit in a case where the rechargeable battery is being charged.
(6) In the liquid medicine administration device according to any one of (1) to (5),
   the pump body may include a disposable cartridge including the reservoir, and a reusable device body including the rechargeable battery, the measurement unit, the notification unit, and the control unit, and
   the control unit may cause the notification unit to provide the notification of the remaining amount of the rechargeable battery measured by the measurement unit in response to coupling of the cartridge to the device body or separation of the cartridge from the device body.
(7) In the liquid medicine administration device according to any one of (1) to (6),
   the pump body may include
   a plunger that is provided in the reservoir and is movable in a longitudinal direction of the reservoir,
   a movable portion that is moved in a movable region to be engaged with the plunger and be able to press the plunger toward a distal end side of the reservoir, and
   a drive portion that moves the movable portion in the movable region.
(8) The liquid medicine administration device according to any one of (1) to (7) may further include
   the cradle device to which the pump body is attachable.

According to the present disclosure, there is provided
(9) a liquid medicine administration system including
the liquid medicine administration device according to any one of (1) to (8); and
a remote controller used for operating the liquid medicine administration device.

According to the present disclosure, there is provided
(10) a control method for a liquid medicine administration device including a pump body that administers a liquid medicine filled in a reservoir into a living body of a patient,
the pump body including
a pump that transfers the liquid medicine filled in the reservoir into the living body of the patient,
a rechargeable battery that supplies power for driving the pump,
a measurement unit that measures a remaining amount of the rechargeable battery,
a detection unit that detects whether or not the pump body is attached to a cradle device to be stuck to a skin of the patient,
a notification unit that notifies a user of information, and
a control unit, the control method including:
   a step of the control unit causing the notification unit to provide a notification of the remaining amount of the rechargeable battery measured by the measurement unit in a case where the detection unit has detected insertion of the pump body into the cradle device or removal of the pump body from the cradle device.

According to the present disclosure, there is provided
(11) a program for controlling a liquid medicine administration device including a pump body that administers a liquid medicine filled in a reservoir into a living body of a patient,
the pump body including
a pump that transfers the liquid medicine filled in the reservoir into the living body of the patient,
a rechargeable battery that supplies power for driving the pump,
a measurement unit that measures a remaining amount of the rechargeable battery,
a detection unit that detects whether or not the pump body is attached to a cradle device to be stuck to a skin of the patient,
a notification unit that notifies a user of information, and
a control unit,
the program causing the control unit to execute a procedure of causing the notification unit to provide a notification of the remaining amount of the rechargeable battery measured by the measurement unit in a case where the detection unit has detected insertion of the pump body into the cradle device or removal of the pump body from the cradle device.

### Advantageous Effects of Invention

According to an embodiment of the present disclosure, it is possible to more effectively notify a user of a remaining amount of a rechargeable battery of a liquid medicine administration device.

### Brief Description of Drawings

[Fig. 1] Fig. 1 is a diagram illustrating an example of a liquid medicine administration system according to an embodiment.
[Fig. 2] Fig. 2 is a perspective view illustrating an example of a liquid medicine administration device in Fig. 1.
[Fig. 3] Fig. 3 is a perspective view illustrating an example of the liquid medicine administration device in a separated state.
[Fig. 4] Fig. 4 is a perspective view illustrating an example of a pump body in Fig. 3.
[Fig. 5] Fig. 5 is a perspective view illustrating an example of the pump body in an exploded state.
[Fig. 6] Fig. 6 is a diagram illustrating an example of a cartridge in a state in which a nut portion is at a non-contact position.
[Fig. 7] Fig. 7 is a diagram illustrating an example of the cartridge in a state in which the nut portion is at a predetermined position.
[Fig. 8] Fig. 8 is a block diagram illustrating an example of a configuration related to control of the liquid medicine administration device in Fig. 1.
[Fig. 9] Fig. 9 is a block diagram illustrating an example of a hardware configuration of a remote controller in Fig. 1.
[Fig. 10] Fig. 10 is a flowchart illustrating an operation example of the liquid medicine administration device in Fig. 1.
[Fig. 11] Fig. 11 is a flowchart illustrating an operation example of the liquid medicine administration device in Fig. 1.

### Description of Embodiments

Hereinafter, an embodiment of the present disclosure will be described with reference to the drawings. In the respective drawings, parts having the same configuration or function are denoted by the same reference sign. In the description of the present embodiment, redundant description of the same part is sometimes appropriately omitted or simplified.

### (Configuration of liquid medicine administration system)

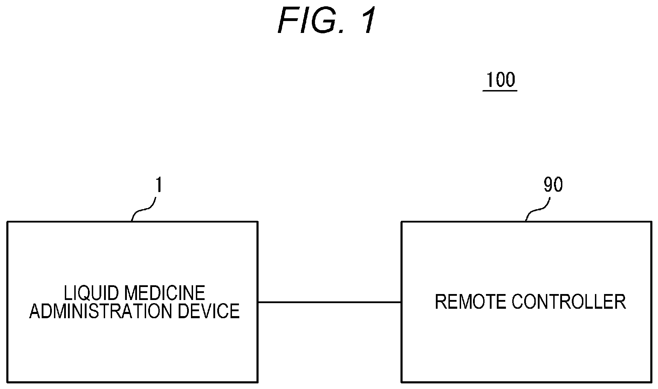

Fig. 1 is a diagram illustrating an example of a liquid medicine administration system 100 according to an embodiment. The liquid medicine administration system 100 administers a liquid medicine such as insulin into a living body of a patient. The liquid medicine administration system 100 includes a liquid medicine administration device 1 and a remote controller 90.

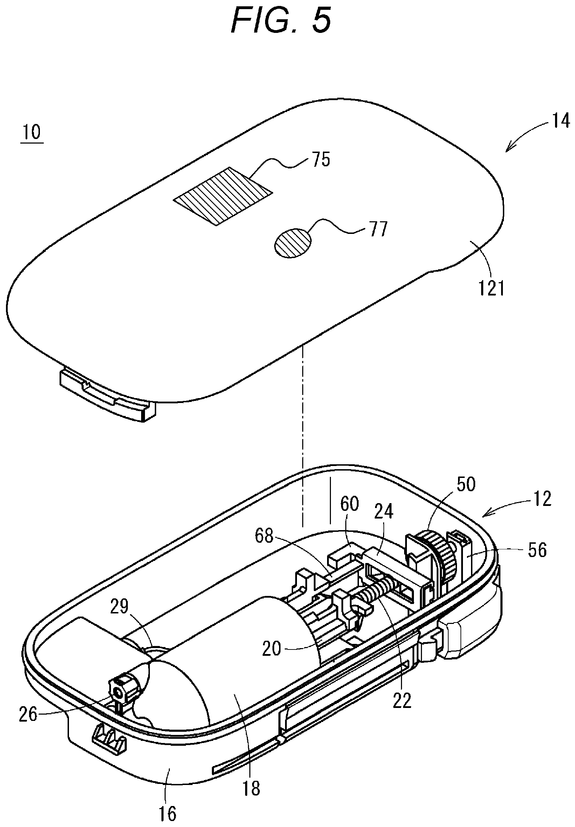

As will be described later with reference to Fig. 5 and the like, the liquid medicine administration device 1 is a device that continuously or intermittently administers a liquid medicine filled in a reservoir (syringe) 18 into a living body by using a pressing action of a plunger 20. The liquid medicine administration device 1 may be, for example, a portable device that can be attached to the abdomen or the like of a patient (patch type). However, the liquid medicine administration device 1 is not limited to the patch type, and may be a tube type or the like.

The remote controller 90 is a device for a user such as a patient to operate the liquid medicine administration device 1. The remote controller 90 notifies the user of the information received from the liquid medicine administration device 1 and receives an operation of the user on the liquid medicine administration device 1. In the present embodiment, the remote controller 90 is realized by a dedicated device corresponding to the liquid medicine administration device 1, but may be realized by a general-purpose information processing device such as a smartphone or a tablet terminal. Furthermore, in the present embodiment, an example in which the remote controller 90 serves as a user interface for notifying a user of information, receiving input of information from the user, and the like will be described, but alternatively, the liquid medicine administration device 1 may include all or some of the functions of the user interface. In the present embodiment, the user who uses the liquid medicine administration system 100 is a patient himself/herself, but the user may be a person other than the patient including a family member of the patient and a medical worker such as a doctor or a nurse.

The liquid medicine administration device 1 and the remote controller 90 are communicatively connected to each other via a wireless communication line, a wired communication line, or a combination thereof. Hereinafter, an example in which the liquid medicine administration device 1 and the remote controller 90 are communicatively connected via Bluetooth (registered trademark) will be described.

### (Configuration of liquid medicine administration device)

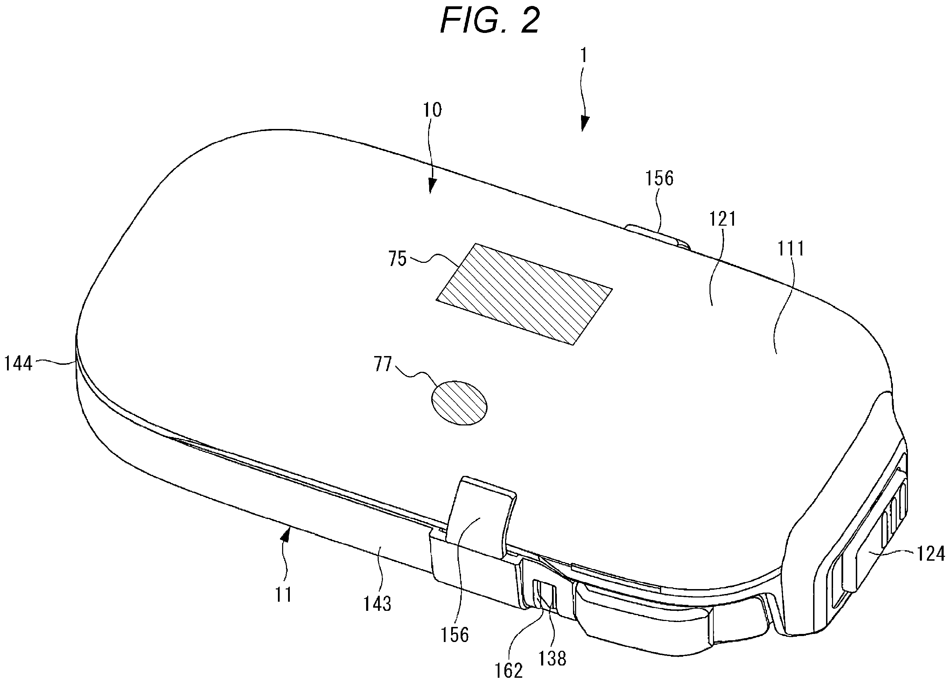

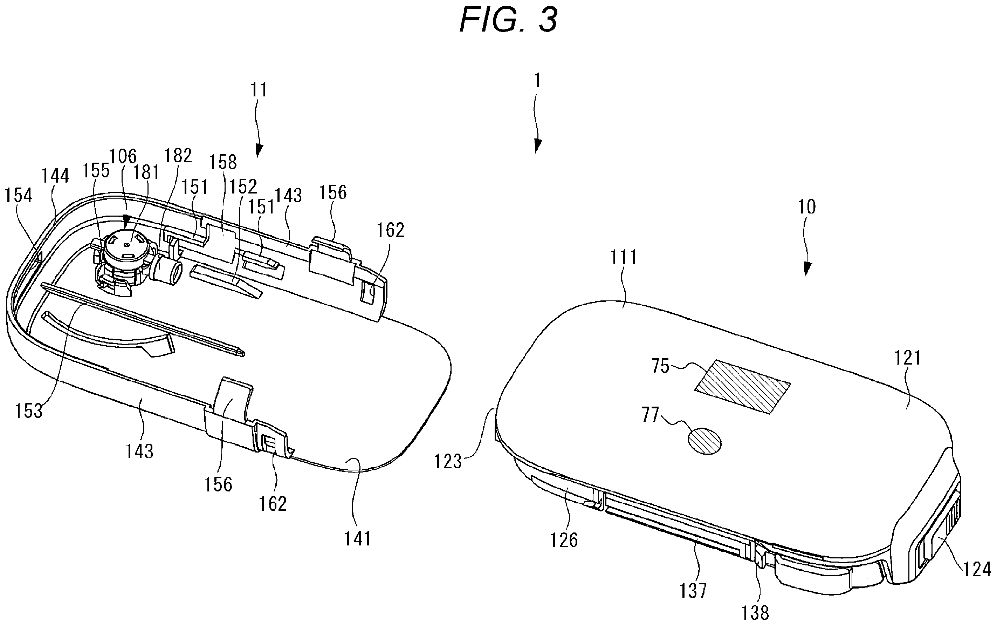

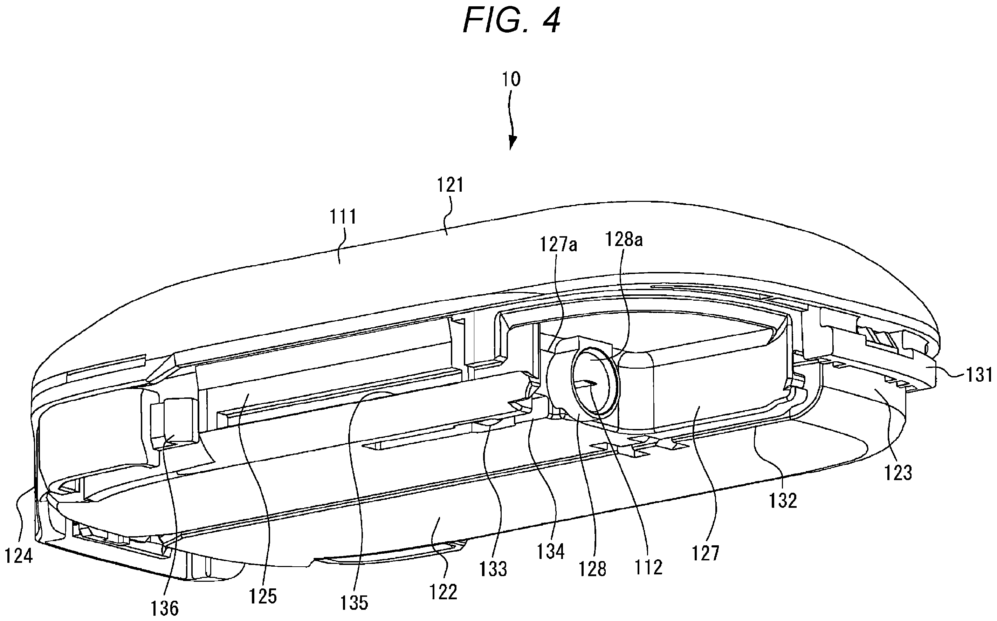

Fig. 2 illustrates an example of a perspective view of the liquid medicine administration device 1 in Fig. 1. Fig. 3 illustrates an example of a perspective view in a case where the liquid medicine administration device 1 in Fig. 1 is separated. The liquid medicine administration device 1 includes a pump body 10, a cradle device 11 to which the pump body 10 is detachably attached, and a cannula port 106 attached to the cradle device 11. The pump body 10 and the cradle device 11 have a repeatedly detachable structure by being engaged with each other. Fig. 4 is a perspective view illustrating an example of the pump body 10 in Fig. 3.

The pump body 10 includes a housing 111 that accommodates constituents of the liquid medicine administration device 1 such as the reservoir 18 and the plunger 20. As exemplified in Figs. 2 and 3 and the like, the housing 111 may be formed in a substantially rectangular parallelepiped shape having a flat shape with curved corners. An upper surface portion 121 of the housing 111 is one surface of the pump body 10 located opposite to the side attached to the cradle device 11. The upper surface portion 121 may be formed in a substantially rectangular shape in which corner portions are curved in top view. A front surface portion 123 and a back surface portion 124 opposing each other are substantially vertically continuous at end portions of the upper surface portion 121 in the first direction. A first side surface portion 125 and a second side surface portion 126 opposing each other are substantially vertically continuous at the end portions of the upper surface portion 121 in the second direction.

As illustrated in Figs. 2 and 3, the pump body 10 includes a connector unit 75 and a notification unit 77 on the upper surface portion 121. The connector unit 75 is a connector to which a charging cable connected to an external power supply can be connected. When the charging cable is connected to connector unit 75, the pump body 10 takes in power from a power supply via the charging cable, and charges a rechargeable battery 42 (see Fig. 5) provided in the device body 14. The connector unit 75 is a connector conforming to a general-purpose standard such as a universal serial bus (USB), but may be realized by another method capable of receiving the supply of power. In Figs. 2 and 3, the connector unit 75 is provided on the upper surface portion 121, but may be provided at any position of the pump body 10 (for example, a side surface of the pump body 10). The connector unit 75 may include a cap in order to prevent dust or the like from entering while the charging cable is not connected. In the present embodiment, the connector unit 75 can be connected to a wired charging cable, but may have a configuration for receiving the supply of power by using a wireless power supply method.

The notification unit 77 notifies the user of information. In the present embodiment, the notification unit 77 is, for example, a light emitting diode (LED) lamp, but may be a speaker that outputs sound, a display (for example, a liquid crystal display) that outputs an image, a vibrator, or a combination thereof. In Figs. 2 and 3, the notification unit 77 is provided on the upper surface portion 121, but may be provided at any position of the pump body 10 (for example, a side surface of the pump body 10).

As illustrated in Figs. 3 and 4, the pump body 10 may have an engagement structure in which the pump body 10 and the cradle device 11 are repeatedly detachable at the first side surface portion 125 and the second side surface portion 126. The engagement structure may have, for example, a hook mechanism. Specifically, a first guide groove portion 135 and a first engagement hook portion 136 may be formed in the first side surface portion 125. A second guide groove portion 137 and a second engagement hook portion 138 may be formed in the second side surface portion 126. The first engagement hook portion 136 may be formed closer to the back surface portion 124 than the first guide groove portion 135. The second engagement hook portion 138 may be formed closer to the back surface portion 124 than the second guide groove portion 137. The first engagement hook portion 136 and the second engagement hook portion 138 may be detachably engaged with a pair of engagement receiving portions 162 of the cradle device 11 that will be described later.

The cradle device 11 is configured to be able to carry the pump body 10. As illustrated in Figs. 2 and 3, the cradle device 11 includes a substantially flat plate-shaped placement surface portion 141 and side wall portions 143 and 144. The placement surface portion 141 is formed in a substantially rectangular shape with curved corners in top view. When the pump body 10 is attached to the cradle device 11, a bottom surface portion 122 (see Fig. 4) of the housing 111 of the pump body 10 is placed on the placement surface portion 141.

A detection rail 152, a sliding rail 153, and an attachment portion 155 may be provided on one surface of the placement surface portion 141. The cannula port 106 may be attached to the attachment portion 155. The attachment portion 155 may be provided with an insertion hole through which a cannula of the cannula port 106 is inserted.

The detection rail 152 is a protrusion portion protruding from one surface of the placement surface portion 141. The detection rail 152 is used by the pump body 10 to detect the attachment of the cradle device 11. The thickness of the detection rail 152 from the placement surface portion 141 gradually increases toward the side wall portion 144. The detection rail 152 extends by a predetermined length in parallel to the side wall portion 143. When the pump body 10 is attached to the cradle device 11, the detection rail 152 enters the detection groove portion 134 provided in the pump body 10 and presses an attachment detection switch 133. The pump body 10 detects the attachment of the cradle device 11 on the basis of the pressing of the attachment detection switch 133. That is, the attachment detection switch 133 functions as a detection unit that detects whether or not the pump body 10 is attached to the cradle device 11.

The sliding rail 153 extends in parallel to the side wall portion 143 on one surface of the placement surface portion 141. When the pump body 10 is attached to the cradle device 11, the sliding groove portion 132 provided in the bottom surface portion 122 of the pump body 10 is slidably fitted into the sliding rail 153.

The side wall portion 144 is substantially vertically continuous to the end portion of the placement surface portion 141 in the first direction. The side wall portions 143 facing each other are substantially vertically continuous to the end portions of the placement surface portion 141 in the second direction. In a state in which the pump body 10 is attached to the cradle device 11, the side wall portions 143 face the first side surface portion 125 and the second side surface portion 126 of the housing 111 of the pump body 10. The side wall portion 144 faces the front surface portion 123 of the housing 111.

As illustrated in Fig. 3, the cradle device 11 may have a fitting hole 154 which is an opening portion in the side wall portion 144. When the pump body 10 is attached to the cradle device 11, a fitting protrusion 131 (see Fig. 4) provided on the front surface portion 123 of the pump body 10 may be fitted into the fitting hole 154.

A guide rail 151, posture correcting portions 156, and engagement receiving portions 162 may be formed on the side wall portions 143. The engagement receiving portions 162 may be opening portions obtained by cutting out the side wall portions 143 in a substantially rectangular shape. The engagement receiving portions 162 may be provided in the side wall portions 143 to face each other according to the arrangement of the first engagement hook portion 136 and the second engagement hook portion 138. When the pump body 10 is attached to the cradle device 11, the first engagement hook portion 136 and the second engagement hook portion 138 may be detachably engaged with the engagement receiving portions 162.

As illustrated in Fig. 3, the guide rail 151 is a protrusion portion formed in the side wall portion 143. In the guide rail 151, the protrusion portion does not necessarily extend continuously. For example, as illustrated in Fig. 3, a notch 158 may be appropriately provided in the middle of the guide rail 151. The guide rail 151 may be provided for each of the side wall portions 143 facing each other. When the pump body 10 is attached to the cradle device 11, the guide rail 151 is engaged with the first guide groove portion 135 provided on the first side surface portion 125 and the second guide groove portion 137 provided on the second side surface portion 126 of the pump body 10. As a result, the attachment direction of the pump body 10 is guided.

As illustrated in Figs. 2 and 3, the posture correcting portions 156 are plate-shaped protruding portions extending upward from the side wall portions 143. The posture correcting portions 156 may each have a curved shape corresponding to the shape of the connection portion (corner portion) between the upper surface portion 121 of the housing 111 of the pump body 10 and the first side surface portion 125 and the second side surface portion 126.

The cradle device 11 may be provided with a sticking sheet to be stuck to the skin of a patient. The sticking sheet may be attached to the other surface opposite to the one surface of the placement surface portion 141 of the cradle device 11. An opening portion through which a cannula of the cannula port 106 that will be described later passes may be formed in the sticking sheet. The sticking sheet may be formed of a flexible member. An adhesive layer to be stuck to the skin of the patient via the sticking sheet may be formed on the surface opposite to the placement surface portion 141. In a state before being stuck to the skin of the patient, an adhesive layer of the sticking sheet may be covered with release paper.

The cannula port 106 may have a port body 181 capable of holding the cannula therein. The port body 181 may have a tubular connection portion. When the cannula is connected, the inside (cylindrical hole) of the connection portion, the port body 181, and the cannula communicate with each other. A cap 182 is attached to the distal portion of the connection portion, and the other end of the connection portion is connected to the port body 181. The cap 182 seals the distal end opening of the connection portion. Accordingly, the inside of the cannula port 106 is separated from the external environment.

When the cannula port 106 is attached to the attachment portion 155 of the cradle device 11 by using a puncture mechanism (not illustrated), the cannula penetrates the placement surface portion 141 together with a puncture needle and protrudes to the other surface (the surface to be stuck to the skin) of the placement surface portion 141. The cannula is punctured into the living body together with the puncture needle. Thereafter, by removing the puncture needle, the cannula is indwelled in the living body.

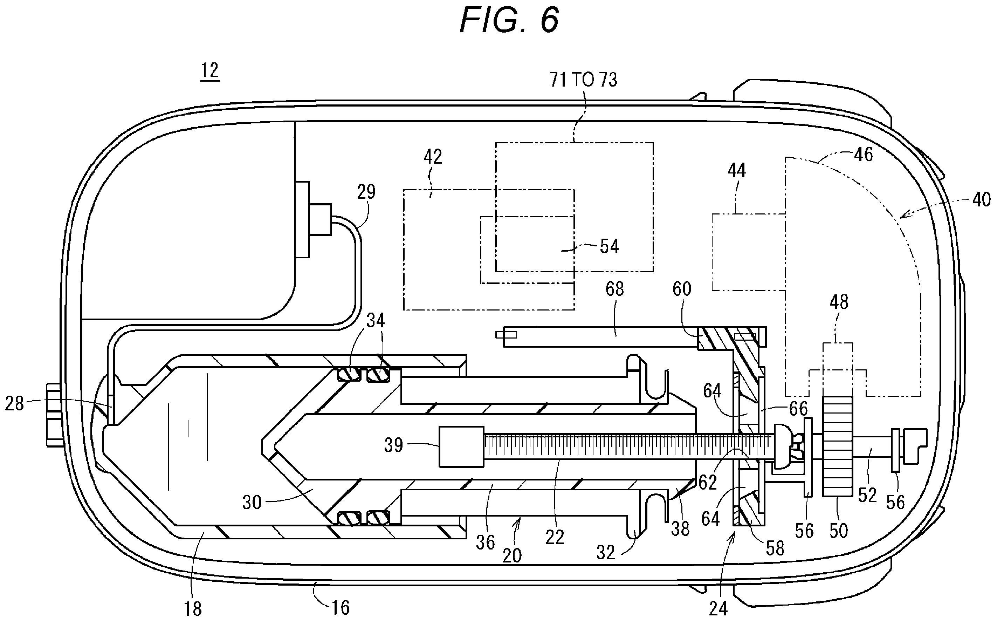

The connection portion of the port body 181 may face the upstream side in the attachment direction. A connection needle tube 112 (see Fig. 4) exposed to the outside of the pump body 10 is fluidly connected to a lead-out tube 29. The connection needle tube 112 enters the cylindrical hole by puncturing the septum surface of the cap 182. As a result, the port body 181 and the lead-out tube 29 (see Fig. 6 and the like) of the pump body 10 are connected, and the lead-out tube 29 and the cannula are fluidly connected. The liquid medicine stored in the reservoir 18 of the pump body 10 is sent out to the cannula port 106 via the lead-out tube 29 and administered from the cannula to the patient through driving of a drive portion 40 (see Fig. 6 and the like). That is, the lead-out tube 29 is connected to the reservoir 18 and functions as a flow path for leading out the liquid medicine to the outside of the reservoir 18. When the connection needle tube 112 is connected to the lead-out tube 29, the lead-out tube 29 and the connection needle tube 112 may be included in the flow path of the liquid medicine administration device 1.

Fig. 4 illustrates a perspective view of an example of the pump body 10 when the pump body 10 is viewed from a direction in which the bottom surface portion 122, the front surface portion 123, and the first side surface portion 125 are visible. A bottom surface storage portion 127 is formed at a corner portion of the bottom surface portion 122, the first side surface portion 125, and the front surface portion 123 in the housing 111. The bottom surface storage portion 127 is a space recessed from the bottom surface portion 122 toward the upper surface portion 121 by a predetermined length (thickness) and a predetermined area (width).

A tubular connection port 128 is formed on a wall surface 127a of the bottom surface storage portion 127 on the back surface portion 124 side in the first direction. The connection port 128 has a cylindrical hole 128a protruding from the wall surface 127a toward the front surface portion 123 side in the first direction (that is, in the attachment direction). The connection needle tube 112 is disposed in the cylindrical hole 128a of the connection port 128. The cylindrical hole 128a protrudes from the wall surface 127a to surround the periphery of the connection needle tube 112, thereby protecting the connection needle tube 112. In addition, the connection needle tube 112 protrudes from the wall surface 127a toward the front surface portion 123 side in the first direction (in the attachment direction) in the cylindrical hole 128a. That is, the direction in which the connection port 128 protrudes from the wall surface 127a is parallel to the first direction.

When the pump body 10 is attached to the cradle device 11, the cannula port 106 attached to the cradle device 11 is accommodated in the bottom surface storage portion 127. Further, the port body 181 and the cap 182 of the cannula port 106 are inserted into the cylindrical hole 128a of the connection port 128. In addition, the connection needle tube 112 protrudes from the wall surface 127a toward the front surface portion 123 side in the first direction (that is, in the attachment direction) in the cylindrical hole 128a. That is, the direction in which the connection needle tube 112 protrudes from the wall surface 127a is parallel to the first direction.

### (Configuration of pump body)

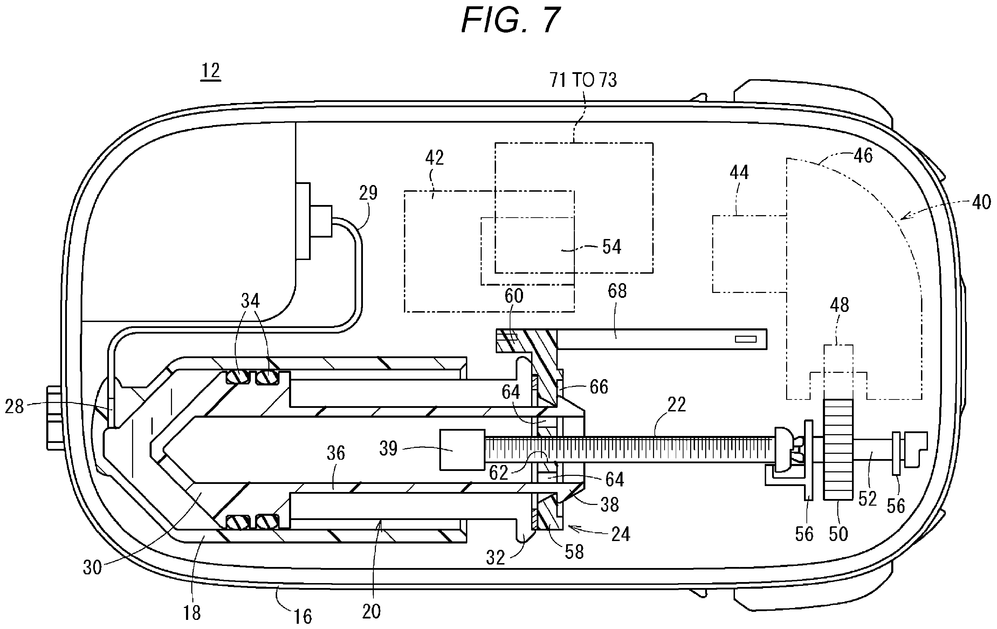

Fig. 5 is an example of an exploded perspective view of the pump body 10 in Fig. 3. Fig. 6 is a diagram illustrating an example of the cartridge 12 in a state in which the nut portion 24 is at a non-contact position. Fig. 7 is a diagram illustrating an example of the cartridge 12 in a state in which the nut portion 24 is at a predetermined position.

As illustrated in Fig. 5, the pump body 10 includes a disposable cartridge 12 and a reusable device body 14. The cartridge 12 includes a base portion 16 having a flat box shape with one side open. The base portion 16 has a substantially rectangular shape in plan view. The base portion 16 may be detachably provided with respect to the cradle device 11 that can be attached to the skin of the patient.

As illustrated in Fig. 5, the base portion 16 is provided with a reservoir 18 filled with a liquid medicine, a plunger 20 provided in the reservoir 18, a feed screw shaft 22 disposed coaxially with the plunger 20, and a nut portion (movable portion) 24 screwed to a feed screw shaft 22. The base portion 16 is configured as a disposable member.

The reservoir 18 extends in a cylindrical shape in the longitudinal direction of the base portion 16. The distal portion of the reservoir 18 has an outer diameter and an inner diameter reduced toward the distal end. An introduction port 26 for introducing a liquid medicine into the reservoir 18 and the lead-out port 28 (see Fig. 6) for leading out the liquid medicine in the reservoir 18 are formed at the distal portion of the reservoir 18. A lead-out tube 29 that leads the liquid medicine in the reservoir 18 to the cannula communicates with the lead-out port 28.

As illustrated in Fig. 6, the plunger 20 is integrally made of a resin material or the like, and is provided in the reservoir 18 to be liquid-tightly slidable in the axial direction of the reservoir 18. The plunger 20 includes a plunger body 30 configuring a distal end side, and a pusher 32 provided on the plunger body 30 and configuring a rear end side. A seal member (sealing member) 34 is attached to the rear end side formed in a cylindrical shape of the plunger body 30. The seal member 34 is attached to the outer surface of the plunger body 30. The seal member 34 is in pressure contact with the inner wall surface of the reservoir 18 to prevent leakage of the liquid medicine filled in the reservoir 18. The seal member 34 moves along the inner wall surface of the reservoir 18 forward and backward in the left-right direction in Figs. 6 and 7 while being fitted to the cylindrical inner wall surface such that the liquid medicine does not leak from the boundary between the plunger body 30 and the inner wall surface of the reservoir 18. The size of the internal space of the reservoir 18 accommodating the liquid medicine varies depending on the position of the plunger body 30 in the reservoir 18. In the present embodiment, the seal member 34 is formed of an O-ring, but the present invention is not limited thereto as long as the liquid medicine in the reservoir 18 can be prevented from leaking from the plunger body 30. The seal member 34 may be made of an elastic material such as silicone rubber.

The pusher 32 includes a pair of extending portions 36 extending rearward from the plunger body 30 to the outside of the reservoir 18, and a pair of claw portions 38 provided at the rear end portions of the extending portions 36. One end portion of the feed screw shaft 22 is pivotally supported by a bearing 39, and configures a drive portion 40 that moves the nut portion 24 in the movable region.

The drive portion 40 further includes a rechargeable battery 42 as a power source, a motor 44 driven by the rechargeable battery 42, a gear box (power transmission mechanism) 46 that decelerates and transmits the rotational driving force of the motor 44, and a transmission shaft 52 to which a spur gear 50 meshing with an output gear 48 of the gear box 46 is fixed and which is integrally rotatably locked to the feed screw shaft 22.

In the present embodiment, the transmission shaft 52 is provided in the cartridge 12, and the rechargeable battery 42, the motor 44, and the gear box 46 are provided in the device body 14. By providing the rechargeable battery 42, the motor 44, and the gear box 46 in the device body 14 as described above, the cost of the cartridge 12 can be reduced.

The rechargeable battery 42 is a secondary battery capable of repeating charging and discharging. The rechargeable battery 42 is provided with a terminal 54 that is electrically connected to the motor 44 of the device body 14 when the device body 14 is connected to the cartridge 12. The transmission shaft 52 is pivotally supported by a pair of bearings 56 provided on the base portion 16 in a state of being disposed coaxially with the feed screw shaft 22.

When the motor 44 rotates, a rotational force thereof is transmitted to the feed screw shaft 22, and the nut portion 24 is moved in a direction toward the reservoir 18 or a direction away from the reservoir 18 due to a rotational action of the feed screw shaft 22. Hereinafter, the rotation of the motor 44 for moving the nut portion 24 in the direction toward the reservoir 18 will be referred to as rotation in the forward direction (forward rotation). Rotation in a direction opposite to the forward direction will be referred to as rotation in the reverse direction (reverse rotation). The motor 44 is configured to be able to rotate both in the forward direction and in the reverse direction. The motor 44 is configured such that the rotational driving force is not transmitted to the constituents of the gear box 46 and the subsequent portions when a certain force or more is applied to the rotation in the forward direction or the rotation in the reverse direction. For example, a stepping motor may be adopted as the motor 44. In a case where the stepping motor is adopted, when a certain force or more is applied to the rotation in the forward direction or the rotation in the reverse direction, the motor 44 is not synchronized with the input pulse, and thus the rotational driving force is not transmitted (step-out). Even when the motor 44 rotates in the reverse direction in a state in which the nut portion 24 is in contact with the bearing 56 side due to step-out, it is possible to prevent damage due to application of an excessive force to the mechanisms such as the motor 44, the gear box 46, and the transmission shaft 52. A rotary encoder (not illustrated) is provided on the output shaft of the motor 44, and it is possible to determine that the motor 44 is not synchronized with the input pulse (step-out) by the rotary encoder detecting the rotation of the motor 44. The operation state of the motor 44 is transmitted to a control unit 71 as a rotary encoder output.

The nut portion 24 is integrally made of a resin material, and includes a nut portion body 58 formed in a substantially rectangular parallelepiped shape, and a slide portion 60 provided on the nut portion body 58. In the nut portion body 58, a screw hole 62 into which the feed screw shaft 22 is screwed and a pair of through holes 64 formed to sandwich the screw hole 62 from both sides and through which the claw portion 38 is inserted are formed. For example, a reinforcing cover 66 made of a metal material or the like is attached to the outer surface of the nut portion body 58.

The slide portion 60 slides with respect to a guide wall 68 provided on the base portion 16 and extending in the axial direction of the plunger 20. That is, the nut portion 24 is at a non-contact position not in contact with the plunger 20 in a state before use (see Fig. 6), and is moved from the non-contact position to a contact position where the nut portion 24 and the plunger 20 are locked due to the rotational action of the feed screw shaft 22. When the feed screw shaft 22 further rotates after contact with the plunger 20, the nut portion 24 presses the plunger 20 toward the distal end side (see Fig. 7). A restricting portion serving as a stopper may be provided on the guide wall 68 so that the slide portion 60 cannot further retreat.

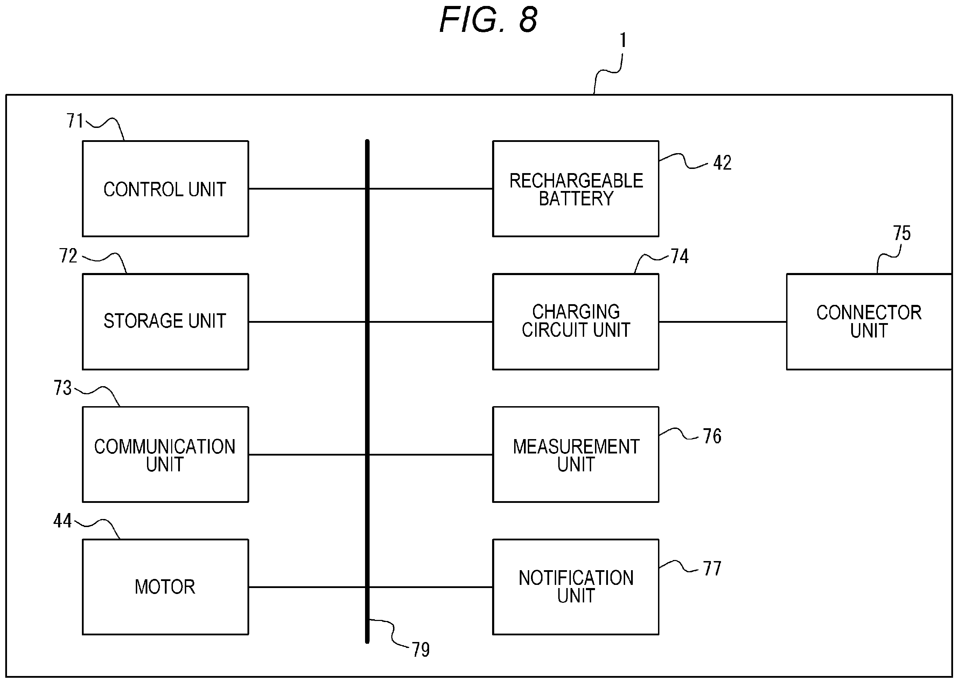

As illustrated in Figs. 5 to 7, the device body 14 includes a lid detachably provided on the base 16 to close the opening of the base portion 16, a control unit 71, a storage unit 72, and a communication unit 73. The lid may be provided on the upper surface portion 121 of the housing 111. To the control unit 71, the rechargeable battery 42, the motor 44, a storage unit 72, a communication unit 73, a charging circuit unit 74, the connector unit 75, a measurement unit 76, and the notification unit 77 are electrically connected via a bus 79 (see Fig. 8). The control unit 71 controls each unit of the liquid medicine administration device 1 to execute processing related to the operation of the liquid medicine administration device 1. For example, the control unit 71 drives and controls the motor 44 on the basis of information regarding liquid medicine administration transmitted from the remote controller 90.

Fig. 8 is a block diagram illustrating an example of a configuration related to control of the liquid medicine administration device 1 in Fig. 1. As described above, the rechargeable battery 42, the motor 44, the storage unit 72, the communication unit 73, the charging circuit unit 74, the connector unit 75, the measurement unit 76, and the notification unit 77 are electrically connected to the control unit 71 via the bus 79.

The control unit 71 is one or more processors. The control unit 71 is communicatively connected to each constituent configuring the liquid medicine administration device 1 and controls the operation of the entire liquid medicine administration device 1. The processor is a general-purpose processor such as a central processing unit (CPU) or a dedicated processor specialized for specific processing. The control unit 71 may include one or more dedicated circuits, or one or more processors may be replaced with one or more dedicated circuits in the control unit 71. The dedicated circuit is, for example, a field programmable gate array (FPGA).

The storage unit 72 is one or more semiconductor memories, one or more magnetic memories, one or more optical memories, or a combination of at least two of these. The semiconductor memory is, for example, a random access memory (RAM) or a read only memory (ROM). The storage unit 72 functions as, for example, a main storage device, an auxiliary storage device, or a cache memory. For example, the storage unit 72 may store information from the control unit 71. Furthermore, the information stored in the storage unit 72 may be transmitted to the remote controller 90 via the communication unit 73 under the control of the control unit 71.

The communication unit 73 is a communication interface for communicating with the remote controller 90. In the present embodiment, the communication unit 73 communicates with the remote controller 90 through Bluetooth (registered trademark), but the present invention is not limited thereto, and for example, communication may be performed via another wireless communication path such as a wireless local area network (LAN) or a wired cable.

The charging circuit unit 74 is an electric circuit for transferring power supplied from a power supply connected to connector unit 75 to rechargeable battery 42. The charging circuit unit 74 includes a switch capable of switching between electrical connection and disconnection between the connector unit 75 and the rechargeable battery 42.

The measurement unit 76 measures a remaining amount (remaining capacity) of the rechargeable battery 42. The measurement unit 76 may measure the remaining amount of the rechargeable battery 42 by using any method such as a voltage measurement method, a coulomb counter method, a battery cell modeling method, or an impedance track method.

The control of the liquid medicine administration device 1 may be executed by the processor included in the control unit 71 executing a program. That is, the control of the liquid medicine administration device 1 may be realized by software. In this case, the program causes a computer to execute processing in a step included in the operation of the liquid medicine administration device 1, thereby causing the computer to realize a function corresponding to the processing in the step. Alternatively, some or all of the functions of the liquid medicine administration device 1 may be realized by a dedicated circuit included in the control unit 71. That is, some or all of the functions of the liquid medicine administration device 1 may be realized by hardware.

### (Configuration of remote controller)

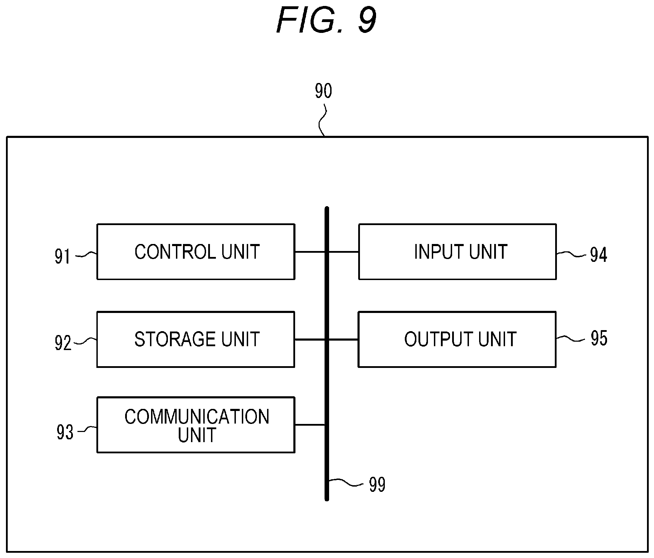

Fig. 9 is a block diagram illustrating an example of a hardware configuration of the remote controller 90 in Fig. 1. The remote controller 90 includes a control unit 91, a storage unit 92, a communication unit 93, an input unit 94, an output unit 95, and a bus 99.

The control unit 91 is one or more processors. The control unit 91 is communicatively connected to each constituent configuring the remote controller 90 via the bus 99, and controls the entire operation of the remote controller 90. The processor is a general-purpose processor such as a CPU or a graphics processing unit (GPU), or a dedicated processor specialized for specific processing. The control unit 91 may include one or more dedicated circuits, or one or more processors may be replaced with one or more dedicated circuits in the control unit 91. The dedicated circuit is, for example, an FPGA.

The storage unit 92 is one or more semiconductor memories, one or more magnetic memories, one or more optical memories, or a combination of at least two of these. The semiconductor memory is, for example, a RAM or a ROM. The RAM is, for example, a static RAM (SRAM) or a dynamic RAM (DRAM). The ROM is, for example, an electrically erasable programmable ROM (EEPROM). The storage unit 92 functions as, for example, a main storage device, an auxiliary storage device, or a cache memory.

The communication unit 93 is a communication interface for communicating with the liquid medicine administration device 1. The communication unit 93 communicates with the liquid medicine administration device 1 to transmit information input by the user to the liquid medicine administration device 1 or receive information from the liquid medicine administration device 1. The communication unit 93 communicates with the liquid medicine administration device 1 through, for example, Bluetooth (registered trademark), but is not limited thereto, and may communicate via, for example, another wireless communication path such as a wireless LAN or a wired cable.

The input unit 94 includes one or more input interfaces that receive a user's input operation and acquire input information based on the user's operation. The input unit 94 is, for example, a touch screen provided integrally with a display (display device) of the output unit 95, but is not limited thereto, and may be a physical key (for example, an external numeric keypad), a capacitance key, a pointing device, a microphone for receiving voice input, or the like.

The output unit 95 as a display unit includes one or more output interfaces that output information to the user and notify the user. For example, the output unit 95 is a display, an LED, a speaker, a vibrator, or the like that outputs information by image display, but is not limited thereto. The input unit 94 and the output unit 95 act as an input/output unit that is an interface between the user and the liquid medicine administration device 1. In the present embodiment, an example in which such an input/output unit is provided in the remote controller 90 will be described, but the input/output unit may be provided in the liquid medicine administration device 1 or another device.

The function of the remote controller 90 may be realized by the processor included in the control unit 91 executing the program according to the present embodiment. That is, the functions of the remote controller 90 may be realized by software. In this case, the program causes the computer to execute processing in a step included in the operation of the remote controller 90, thereby causing the computer to realize a function corresponding to the processing in the step. Alternatively, some or all of the functions of the remote controller 90 may be realized by a dedicated circuit included in the control unit 91. That is, some or all of the functions of the remote controller 90 may be realized by hardware.

### (Operation of liquid medicine administration device)

When operating the liquid medicine administration device 1 according to the present embodiment, first, the user takes out the cartridge 12 from a packaging container. In this state, the reservoir 18 of the cartridge 12 is not filled with the liquid medicine, and the nut portion 24 is at a non-contact position not in contact with the plunger 20 (see Fig. 6).

Next, the user adjusts the position of the plunger 20 with respect to the reservoir 18, and fills any amount of the liquid medicine from the introduction port 26 into the reservoir 18 from a liquid medicine container such as a vial in which the liquid medicine is hermetically stored. Thereafter, the user connects the device body 14 to the cartridge 12. As a result, the power of the rechargeable battery 42 of the cartridge 12 is supplied to the constituents of the device body 14, and the output gear 48 of the gear box 46 of the device body 14 meshes with the spur gear 50 of the cartridge 12. The control unit 71, the storage unit 72, and the like are activated by the supply of power from the rechargeable battery 42.

Subsequently, the user fills the cartridge 12 taken out of the packaging container with the liquid medicine, connects the cartridge 12 and the device body 14, and then performs priming of the liquid medicine administration device 1. The priming is an operation of locking the nut portion 24 of the liquid medicine administration device 1 to the plunger 20 and filling the flow path of the liquid medicine administration device 1 including the lead-out tube 29 with the liquid medicine. Specifically, the user operates the remote controller 90 to rotationally drive the motor 44 in the forward direction. As a result, since the rotational driving force of the motor 44 is transmitted to the feed screw shaft 22 via the gear box 46, the spur gear 50, and the transmission shaft 52, the feed screw shaft 22 rotates, and the nut portion 24 advances toward the plunger 20 while sliding on the guide wall 68.

When the nut portion 24 advances toward the distal end side of the reservoir 18, the pair of claw portions 38 abuts on the wall surfaces configuring the through holes 64 of the nut portion 24, and the pair of extending portions 36 is bent to approach each other. When the claw portions 38 pass through the through holes 64, the extending portions 36 return from the bent state to the original state, and the nut portion 24 is locked to the rear end portion of the plunger 20. As a result, the nut portion 24 can press the plunger 20 toward the distal end side. Thereafter, by further advancing the nut portion 24, the liquid medicine in the reservoir 18 is pressed by the plunger 20, the inner hole of the lead-out tube 29 is filled with the liquid medicine, and the priming is completed. This priming is completed when the user visually recognizes that the liquid medicine is discharged from the connection needle tube 112 fluidly connected to the lead-out tube 29 and exposed to the outside of the liquid medicine administration device 1. When the user visually recognizes that the liquid medicine has been discharged from the connection needle tube 112, the user instructs the liquid medicine administration device 1 to stop the priming. For example, the remote controller 90 may display an image of a priming stop button on the display of the output unit 95 and notify the liquid medicine administration device 1 of the stop of the motor 44 in response to the selection of the priming stop button by the user.

Subsequently, the user sticks the cradle device 11 to a predetermined position of the skin (for example, the abdomen of the patient), indwells the cannula of the cannula port 106 in the living body by using the puncture mechanism, and locks the cannula port 106 to the cradle device 11. Next, when the user attaches, to the cradle device 11, the pump body 10 in which the cartridge 12 and the device body 14 are connected, the lead-out tube 29 and the cannula communicate with each other. When the control unit 71 controls the rotation of the motor 44 in this state, the liquid medicine in the reservoir 18 is continuously or intermittently administered into the living body. The control unit 71 controls the rotation of the motor 44 according to the schedule of liquid medicine administration for which an instruction is given from the remote controller 90, and administers the liquid medicine at various rates such as a basal rate (basal) or a bolus according to the patient's condition. The basal rate is the amount of the liquid medicine per unit time corresponding to basal secretion of insulin. The bolus is the amount of the liquid medicine corresponding to additional secretion of insulin with respect to a meal or an increase in blood glucose level.

The reservoir 18 of the cartridge 12 is filled with an amount of liquid medicine to be administered in an administration cycle of a certain number of days. The liquid medicine filled in the reservoir 18 is administered into the living body over, for example, 3 days to 1 week, and then the cartridge 12 is replaced and discarded. Every one administration cycle, the cartridge 12 is replaced with a new cartridge. Each time the cartridge 12 is replaced, filling of the reservoir 18 of the cartridge 12 with the liquid medicine, connection between the cartridge 12 and the device body 14, and a priming operation are performed. Through such operations, while the liquid medicine is administered, the liquid medicine administration device 1 can estimate the liquid medicine administration amount on the basis of, for example, the rotation speed of the motor 44 performed in the priming and the liquid delivery. Since the liquid medicine administration device 1 according to the present embodiment includes the disposable cartridge 12 and the reusable device body 14, the running cost can be reduced.

The amount of the liquid medicine filled in the reservoir 18 of the cartridge 12 varies depending on the age, the condition, and the like of the patient even if the length of the administration cycle is the same. For example, in a case where the reservoir 18 is filled with a liquid medicine for three days, the adult cartridge 12 is filled with a larger amount of liquid medicine than the pediatric cartridge 12.

As described above, the liquid medicine administration device 1 according to the present embodiment includes the rechargeable battery 42 capable of repeating charging and discharging as a power source. The rechargeable battery 42 is provided in the reusable device body 14. Therefore, the liquid medicine administration device 1 needs to notify the user of the remaining amount of the rechargeable battery 42 at an appropriate timing and notify the user of the fact that charging is necessary so that the device does not stop in the middle of liquid medicine administration.

Therefore, the liquid medicine administration device 1 causes the notification unit 77 to notify the user of the remaining amount of the rechargeable battery 42 measured by the measurement unit 76 in response to detection of insertion of the pump body 10 into the cradle device 11 or removal of the pump body 10 from the cradle device 11. In general, when performing work of inserting and removing the pump body 10 into and from the cradle device 11, the user often looks at the liquid medicine administration device 1. The liquid medicine administration device 1 notifies the user of the remaining amount of the rechargeable battery 42 from the notification unit 77 in a situation in which the user's eyes are easily directed as described above, so that the user can be more effectively notified of the remaining amount of the rechargeable battery 42.

In a case where the notification unit 77 includes one LED lamp, the notification unit 77 may notify the user of the remaining amount of the rechargeable battery 42 by using a color of the LED lamp. For example, in a case where the remaining amount of rechargeable battery 42 exceeds a certain value, the notification unit 77 may indicate the remaining battery amount in white, blue, green, or the like. Alternatively, for example, in a case where the remaining amount of the rechargeable battery 42 is less than a certain value, the notification unit 77 may indicate the remaining battery amount in red, orange, or the like. Alternatively, in a case where the remaining amount of the rechargeable battery 42 is small and the necessity of charging the rechargeable battery 42 is particularly high, the notification unit 77 may blink light of a predetermined color. In addition, in a case where the notification unit 77 includes a plurality of LED lamps, the notification unit 77 may indicate the remaining amount of the rechargeable battery 42 by using the number of LED lamps to be turned on. In a case where the notification unit 77 includes a display, the notification unit 77 may indicate the remaining amount of rechargeable battery 42 by displaying a numerical value, a graph, or the like. In a case where the notification unit 77 includes a speaker or a vibrator, the notification unit 77 may indicate the necessity of charging by using sound or vibration together with an LED lamp or a display. In a case where the notification unit 77 provides a notification by using voice, a tone or a scale may be changed according to the remaining amount of the rechargeable battery 42. In addition, depending on the surrounding situation, the notification unit 77 may provide a notification in a non-auditory manner using at least one of an LED lamp, a display, or a vibrator.

In addition, in a case where the pump body 10 is inserted into the cradle device 11, the liquid medicine administration device 1 may cause the notification unit 77 to notify the user of the remaining amount of the rechargeable battery 42 when a predetermined time has elapsed since the previous charging. In general, in a case where a predetermined time has elapsed since the previous charging, it is considered that the remaining amount of the rechargeable battery 42 is often decreased, and it can be said that there is a high need to check the remaining amount of the rechargeable battery 42. As described above, since the liquid medicine administration device 1 notifies the user of the remaining amount of the rechargeable battery 42 in a case where it is highly necessary to notify the user of the remaining amount of the rechargeable battery 42, it is possible to effectively notify the user of the remaining amount of the rechargeable battery 42.

Further, in a case where the pump body 10 is removed from the cradle device 11, the liquid medicine administration device 1 may cause the notification unit 77 to notify the user of the remaining amount of the rechargeable battery 42 when the remaining amount of the rechargeable battery 42 is less than a certain value. The pump body 10 is removed from the cradle device 11 when the cartridge 12 is replaced, for example. In a case where such a cartridge 12 is replaced, it is generally considered that the remaining amount of the rechargeable battery 42 is reduced after the liquid medicine administration operation is finished. In such a case, when the remaining amount of the rechargeable battery 42 is less than a certain value, it is considered that there is a high necessity for the liquid medicine administration device 1 to notify the user of the remaining battery amount. As described above, since the liquid medicine administration device 1 notifies the user of the remaining amount of the rechargeable battery 42 in a case where it is highly necessary to notify the user of the remaining amount of the rechargeable battery 42, it is possible to effectively notify the user of the remaining amount of the rechargeable battery 42.

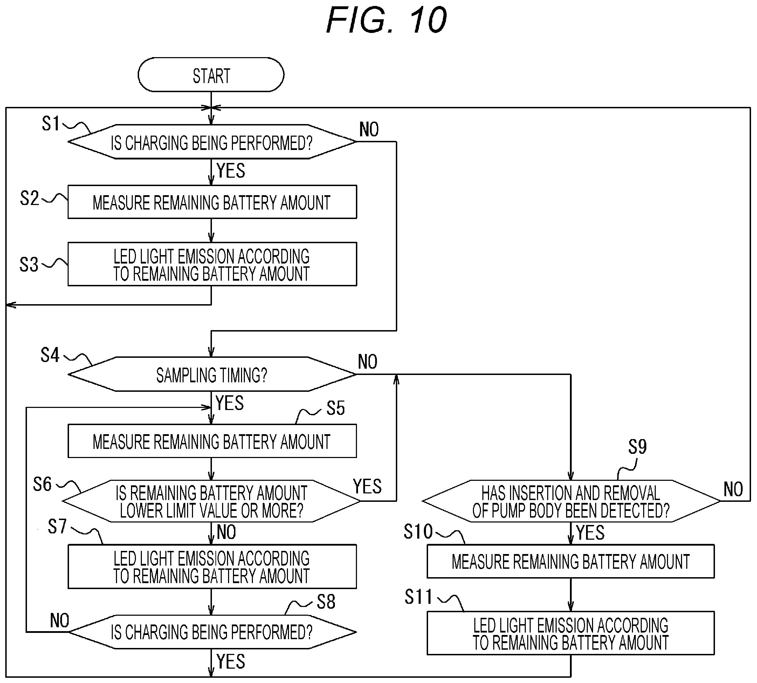

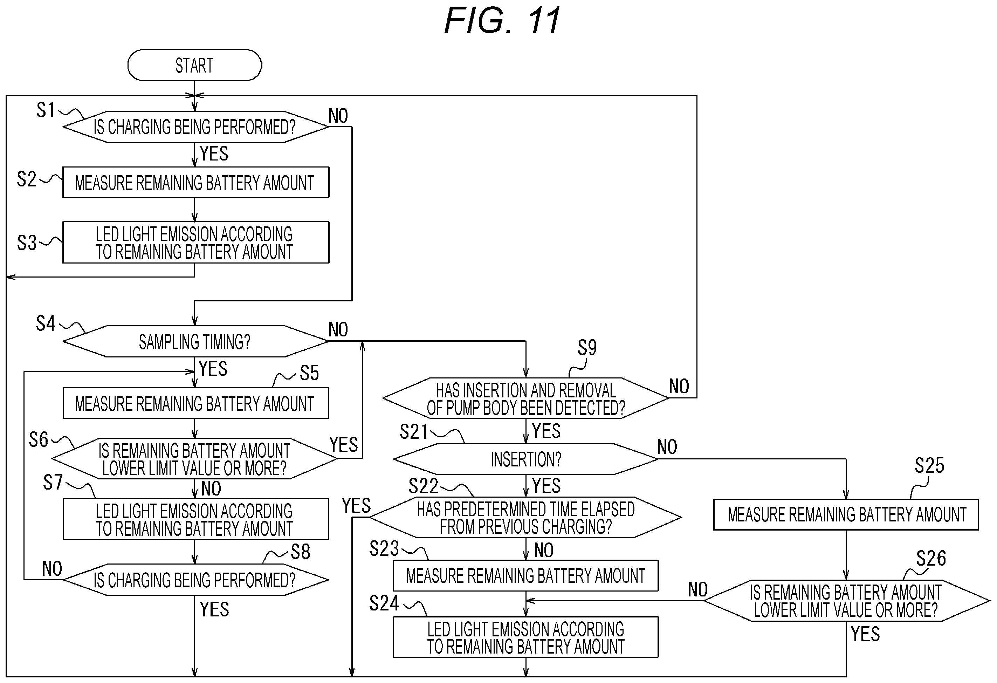

Figs. 10 and 11 are flowcharts illustrating an operation example of the liquid medicine administration device 1 in Fig. 1. The operation of the liquid medicine administration device 1 described with reference to Figs. 10 and 11 may correspond to one of methods of controlling the liquid medicine administration device 1. The operation in each step in Figs. 10 and 11 may be executed on the basis of control of the control unit 71 of the liquid medicine administration device 1.

In steps S1 to S3 in Fig. 10, the control unit 71 performs processing of notifying a user of a remaining battery amount during charging of the rechargeable battery 42.

In step S1, the control unit 71 determines whether or not the rechargeable battery 42 is being charged. Specifically, the control unit 71 may measure a voltage or a current of a part of the charging circuit unit 74, determine whether or not power is supplied to the rechargeable battery 42, and determine whether or not the rechargeable battery 42 is being charged. Alternatively, whether or not the charging terminal is connected may be determined by using a voltage or a current. The control unit 71 proceeds to step S2 in a case where charging is being performed (YES in step S1), and proceeds to step S4 otherwise (NO in step 1).

In step S2, the control unit 71 causes the measurement unit 76 to measure a remaining amount of the rechargeable battery 42.

In step S3, the control unit 71 causes the notification unit 77 to notify the user of the remaining amount of the rechargeable battery 42 according to the remaining amount of the rechargeable battery 42 measured in step S2. A specific example of the remaining amount notification is as described above. When the processing in step S3 is finished, the control unit 71 returns to step S1. As described above, the liquid medicine administration device 1 notifies the user of the remaining battery amount at any time during charging of the rechargeable battery 42. Therefore, the user can charge the rechargeable battery 42 until charging of the rechargeable battery 42 is completed.

In steps S4 to S8, the control unit 71 measures the remaining battery amount of the rechargeable battery 42 at a constant sampling rate (for example, every 10 seconds), and performs processing of notifying the user of the remaining battery amount when the remaining battery amount falls below a predetermined lower limit value.

In step S4, the control unit 71 determines whether or not a sampling timing of the remaining battery amount arrives. For example, in a case where the remaining battery amount is measured every 10 seconds, the control unit 71 determines whether or not the timing of the measurement arrives. The control unit 71 proceeds to step S5 in a case where the sampling timing arrives (YES in step S4), and proceeds to step S9 otherwise (NO in step S4).

In step S5, the control unit 71 causes the measurement unit 76 to measure a remaining amount of the rechargeable battery 42.

In step S6, the control unit 71 determines whether or not the remaining amount of the rechargeable battery 42 measured in step S5 is a predetermined lower limit value or more (threshold). The control unit 71 proceeds to step S9 in a case where the remaining amount is the lower limit value or more (YES in step S6), and proceeds to step S7 otherwise (NO in step S6).

In step S7, the control unit 71 causes the notification unit 77 to notify the user of the remaining amount of the rechargeable battery 42 according to the remaining amount of the rechargeable battery 42 measured in step S5. A specific example of the remaining amount notification is as described above.

In step S8, the control unit 71 determines whether or not the rechargeable battery 42 is being charged. Whether or not the rechargeable battery 42 is being charged can be determined in the same manner as in step S1. The control unit 71 proceeds to step S1 in a case where charging is being performed (YES in step S8), and proceeds to step S5 otherwise (NO in step 8). As described above, the liquid medicine administration device 1 measures the remaining battery amount of the rechargeable battery 42 at a constant sampling rate, and notifies the user of the remaining battery amount in a case where the remaining battery amount falls below a predetermined lower limit value. Therefore, in a case where the necessity of charging the rechargeable battery 42 is high, the user can easily ascertain the high necessity.

In steps S9 to S11, the control unit 71 performs processing of notifying the user of the remaining battery amount of the rechargeable battery 42 in response to insertion and removal of the pump body 10 into and from the cradle device 11.

In step S9, the control unit 71 determines whether or not it is detected that the pump body 10 is inserted into the cradle device 11 or the pump body 10 is removed from the cradle device 11. Such insertion and removal of the pump body 10 into and from the cradle device 11 can be detected by the attachment detection switch 133. The control unit 71 proceeds to step S10 in a case where insertion and removal of the pump body 10 is detected (YES in step S9), and returns to step S1 otherwise (NO in step S9).

In step S10, the control unit 71 causes measurement unit 76 to measure a remaining amount of the rechargeable battery 42.

In step S11, the control unit 71 causes the notification unit 77 to notify the user of the remaining amount of the rechargeable battery 42 according to the remaining amount of the rechargeable battery 42 measured in step S10. A specific example of the remaining amount notification is as described above. When the processing in step S11 is finished, the control unit 71 returns to step S1. As described above, the liquid medicine administration device 1 notifies the user of the remaining battery amount of the rechargeable battery 42 according to insertion and removal of the pump body 10 into and from the cradle device 11. Therefore, the user can effectively ascertain the remaining amount of the rechargeable battery 42.

Fig. 11 illustrates an example of processing in which the notification procedure of the remaining battery amount is different between a case where the pump body 10 is inserted into the cradle device 11 and a case where the pump body 10 is removed from the cradle device 11 in the flowchart in Fig. 10. In Fig. 11, processing contents of steps S1 to S8 are the same as those of steps S1 to S8 in Fig. 10.

In step S9, the control unit 71 determines whether or not it is detected that the pump body 10 is inserted into the cradle device 11 or the pump body 10 is removed from the cradle device 11. Such insertion and removal of the pump body 10 into and from the cradle device 11 can be detected by the attachment detection switch 133. The control unit 71 proceeds to step S21 in a case where insertion and removal of the pump body 10 is detected (YES in step S9), and returns to step S1 otherwise (NO in step S9).

In step S21, the control unit 71 determines whether or not the operation detected in step S9 is insertion of the pump body 10 into the cradle device 11. The control unit 71 proceeds to step S22 in a case where the operation is insertion (YES in step S21), and proceeds to step S25 otherwise (NO in step S21).

In step S22, the control unit 71 determines whether a predetermined time has elapsed since the previous charging of the rechargeable battery 42. That is, the control unit 71 determines whether or not the rechargeable battery 42 is charged in a certain period before pump body 10 is inserted into the cradle device 11. The lapse of the predetermined time from the previous charging of the rechargeable battery 42 corresponds to the fact that the rechargeable battery 42 is not charged in a certain period before pump body 10 is inserted. The control unit 71 proceeds to step S23 in a case where the predetermined time has elapsed from the previous charging of the rechargeable battery 42 (YES in step S22), and returns to step S1 otherwise (NO in step S22).

In step S23, the control unit 71 causes measurement unit 76 to measure a remaining amount of the rechargeable battery 42.

In step S24, the control unit 71 causes the notification unit 77 to notify the user of the remaining amount of the rechargeable battery 42 according to the remaining amount of the rechargeable battery 42 measured in step S23. A specific example of the remaining amount notification is as described above. As described above, in a case where the pump body 10 is inserted into the cradle device 11 and a predetermined time has elapsed since the previous charging of the rechargeable battery 42, the control unit 71 causes the notification unit 77 to notify the user of the remaining amount of the rechargeable battery 42. As described above, in a case where a certain time has elapsed since the previous charging and there is a possibility that the remaining amount of the rechargeable battery 42 is small, the liquid medicine administration device 1 notifies the user of the remaining amount of the rechargeable battery 42, so that the user can be effectively notified of the remaining amount of the rechargeable battery 42. In the present embodiment, charging of the rechargeable battery 42 is executed when the pump body 10 is not attached to the cradle device 11 from a viewpoint of preventing a failure such as electric leakage.

In step S24, the control unit 71 may cause notification unit 77 to notify the user of the remaining amount of the rechargeable battery 42 continuously for a certain time (for example, 10 seconds). In this manner, the liquid medicine administration device 1 can effectively notify the user of the remaining amount of the rechargeable battery 42 by continuously notifying the user of the remaining amount of the rechargeable battery 42 for a certain time.

When the processing in step S24 is finished, the control unit 71 returns to step S1.

In step S25, the control unit 71 causes measurement unit 76 to measure a remaining amount of the rechargeable battery 42.

In step S26, the control unit 71 determines whether or not the remaining amount of the rechargeable battery 42 measured in step S26 is a predetermined lower limit value (threshold) or more. In a case where the remaining amount is the lower limit value or more (YES in step S26), the control unit 71 returns to step S1. Otherwise (NO in step S26), the control unit 71 proceeds to step S24, and causes the notification unit 77 to notify the user of the remaining amount of the rechargeable battery 42 according to the remaining amount of the rechargeable battery 42 measured in step S25. A specific example of the remaining amount notification is as described above. As described above, since the liquid medicine administration device 1 notifies the user of the remaining amount of the rechargeable battery 42 in a case where the pump body 10 is removed from the cradle device 11 and there is a possibility that the remaining amount of the rechargeable battery 42 is small, it is possible to effectively notify the user of the remaining amount of the rechargeable battery 42. In step S24, the control unit 71 may cause notification unit 77 to notify the user of the remaining amount of the rechargeable battery 42 continuously for a certain time (for example, 10 seconds) as in the case where the pump body 10 is inserted into the cradle device 11.

When the processing in step S24 is finished, the control unit 71 returns to step S1.

As described above, the liquid medicine administration device 1 causes the notification unit 77 to notify the user of the remaining amount of the rechargeable battery 42 in response to insertion and removal of the pump body 10 into and from the cradle device 11, or the like. Therefore, according to the liquid medicine administration device 1, it is possible to more effectively notify the user of the remaining amount of the rechargeable battery 42 of the liquid medicine administration device 1.

The liquid medicine administration device 1 may cause the notification unit 77 to notify the user of the remaining amount of the rechargeable battery 42 measured by the measurement unit 76 in response to the fact that the cartridge 12 is coupled to the device body 14 or the fact that the cartridge 12 is separated from the device body 14. As described above, when the user pays attention to the cartridge 12 and the device body 14, the user is notified of the remaining amount of the rechargeable battery 42, so that the user can be more effectively notified of the remaining amount of the rechargeable battery 42. In order to realize such a configuration, the liquid medicine administration device 1 may include a configuration for detecting the presence or absence of coupling between the cartridge 12 and the device body 14.

In addition, in the present embodiment, an example has been described in which the pump body 10 that transfers the liquid medicine into the living body of the patient is configured by a syringe pump, but the principle of liquid medicine administration is not limited to the syringe. For example, the pump body 10 may be a roller pump, a patient controlled analgesia (PCA) pump, or another pump.

The present disclosure is not limited to the above-described embodiments. For example, a plurality of blocks illustrated in the block diagram may be integrated, or one block may be divided. Instead of being chronologically executed according to the description, a plurality of steps illustrated in the flowchart may be executed in parallel or in a different order, depending on the processing capacity of the device that executes each step or as necessary. In addition, modifications can be made without departing from the gist of the present disclosure.

Furthermore, for example, the configuration and operation of the liquid medicine administration device 1 may be distributed to a plurality of computers capable of communicating with each other. Further, for example, some or all of the constituents of the liquid medicine administration device 1 may be provided in another device such as the remote controller 90.

### Reference Signs List

- 1: Liquid medicine administration device
- 10: Pump body
- 11: Cradle device
- 12: Cartridge
- 14: Device body
- 16: Base portion
- 18: Reservoir
- 20: Plunger
- 22: Feed screw shaft
- 24: Nut portion
- 26: Introduction port
- 28: Lead-out port
- 29: Lead-out tube
- 30: Plunger body
- 32: Pusher
- 34: Seal member
- 36: Extending portion
- 38: Claw portion
- 39: Bearing
- 40: Drive portion
- 42: Rechargeable battery
- 44: Motor
- 46: Gearbox
- 48: Output gear
- 50: Spur gear
- 52: Transmission shaft
- 54: Terminal
- 56: Bearing
- 58: Nut portion body
- 60: Slide portion
- 62: Screw hole
- 64: Through hole
- 66: Reinforcing cover
- 68: Guide wall
- 71: Control unit
- 72: Storage unit
- 73: Communication unit
- 74: Charging circuit unit
- 75: Connector unit
- 76: Measurement unit
- 77: Notification unit
- 79: Bus
- 106: Cannula port
- 111: Housing
- 112: Connection needle tube
- 121: Upper surface portion
- 122: Bottom surface portion
- 123: Front surface portion
- 124: Back surface portion
- 125: First side surface portion
- 126: Second side surface portion
- 127: Bottom surface storage portion
- 127a: Wall surface
- 128: Connection port
- 128a: Cylindrical hole
- 131: Fitting protrusion
- 132: Sliding groove portion
- 133: Attachment detection switch
- 134: Detection groove
- 135: First guide groove portion
- 136: First engagement hook portion
- 137: Second guide groove portion
- 138: Second engagement hook portion
- 141: Placement surface portion
- 143: Side wall portion
- 144: Side wall portion
- 151: Guide rail
- 152: Detection rail
- 153: Sliding rail
- 154: Fitting hole
- 155: Attachment portion
- 156: Posture correcting portion
- 158: Notch
- 162: Engagement receiving portion
- 181: Port body
- 182: Cap
- 90: Remote controller
- 91: Control unit
- 92: Storage unit
- 93: Communication unit
- 94: Input unit
- 95: Output unit
- 99: Bus
- 100: Liquid medicine administration system

## Claims

1. A liquid medicine administration device comprising a pump body that administers a liquid medicine filled in a reservoir into a living body of a patient, wherein
the pump body includes
a pump that transfers the liquid medicine filled in the reservoir into the living body of the patient,
a rechargeable battery that supplies power for driving the pump,
a measurement unit that measures a remaining amount of the rechargeable battery,
a detection unit that detects whether or not the pump body is attached to a cradle device to be stuck to a skin of the patient,
a notification unit that notifies a user of information, and
a control unit, and
the control unit causes the notification unit to provide a notification of the remaining amount of the rechargeable battery measured by the measurement unit in a case where the detection unit has detected insertion of the pump body into the cradle device or removal of the pump body from the cradle device.

2. The liquid medicine administration device according to claim 1, wherein
the control unit determines whether or not a predetermined time has elapsed from previous charging of the rechargeable battery in response to detection of the insertion of the pump body into the cradle device, and
causes the notification unit to provide the notification of the remaining amount of the rechargeable battery measured by the measurement unit in a case where it is determined that the predetermined time has elapsed from the previous charging of the rechargeable battery.

3. The liquid medicine administration device according to claim 1, wherein
the control unit determines whether or not the remaining amount of the rechargeable battery measured by the measurement unit is a predetermined threshold or more in response to detection of the removal of the pump body from the cradle device, and
causes the notification unit to provide the notification of the remaining amount of the rechargeable battery measured by the measurement unit in a case where it is determined that the remaining amount of the rechargeable battery is not the predetermined threshold or more.

4. The liquid medicine administration device according to claim 1, wherein
the control unit causes the measurement unit to measure the remaining amount of the rechargeable battery at a predetermined sampling timing, and
causes the notification unit to provide the notification of the remaining amount of the rechargeable battery measured by the measurement unit in a case where the remaining amount of the rechargeable battery measured at the sampling timing is less than a predetermined threshold.

5. The liquid medicine administration device according to claim 1, wherein
the control unit determines whether or not the rechargeable battery is being charged, and
causes the notification unit to provide the notification of the remaining amount of the rechargeable battery measured by the measurement unit in a case where the rechargeable battery is being charged.

6. The liquid medicine administration device according to claim 1, wherein
the pump body includes a disposable cartridge including the reservoir, and a reusable device body including the rechargeable battery, the measurement unit, the notification unit, and the control unit, and
the control unit causes the notification unit to provide the notification of the remaining amount of the rechargeable battery measured by the measurement unit in response to coupling of the cartridge to the device body or separation of the cartridge from the device body.

7. The liquid medicine administration device according to claim 1, wherein
the pump body includes
a plunger that is provided in the reservoir and is movable in a longitudinal direction of the reservoir,
a movable portion that is moved in a movable region to be engaged with the plunger and be able to press the plunger toward a distal end side of the reservoir, and
a drive portion that moves the movable portion in the movable region.

8. The liquid medicine administration device according to claim 1, further comprising the cradle device to which the pump body is attachable.

9. A liquid medicine administration system comprising:
the liquid medicine administration device according to any one of claims 1 to 8; and
a remote controller used for operating the liquid medicine administration device.

10. A control method for a liquid medicine administration device including a pump body that administers a liquid medicine filled in a reservoir into a living body of a patient,
the pump body including
a pump that transfers the liquid medicine filled in the reservoir into the living body of the patient,
a rechargeable battery that supplies power for driving the pump,
a measurement unit that measures a remaining amount of the rechargeable battery,
a detection unit that detects whether or not the pump body is attached to a cradle device to be stuck to a skin of the patient,
a notification unit that notifies a user of information, and
a control unit, the control method comprising:
a step of the control unit causing the notification unit to provide a notification of the remaining amount of the rechargeable battery measured by the measurement unit in a case where the detection unit has detected insertion of the pump body into the cradle device or removal of the pump body from the cradle device.

11. A program for controlling a liquid medicine administration device including a pump body that administers a liquid medicine filled in a reservoir into a living body of a patient,
the pump body including
a pump that transfers the liquid medicine filled in the reservoir into the living body of the patient,
a rechargeable battery that supplies power for driving the pump,
a measurement unit that measures a remaining amount of the rechargeable battery,
a detection unit that detects whether or not the pump body is attached to a cradle device to be stuck to a skin of the patient,
a notification unit that notifies a user of information, and
a control unit,
the program causing the control unit to execute a procedure of causing the notification unit to provide a notification of the remaining amount of the rechargeable battery measured by the measurement unit in a case where the detection unit has detected insertion of the pump body into the cradle device or removal of the pump body from the cradle device.
